# EUROPEAN PATENT SPECIFICATION

(11) **EP 1 974 666 B1**
(45) Date of publication and mention of the grant of the patent: **19.05.2010**
(21) Application number: 08251217.9
(22) Date of filing: 31.03.2008
(51) Int. Cl.: A61B 5/09

(54) **Hand-held spirometer with a detachable flow head**
Handspirometer mit abtrennbarem Flusskopf
Spiromètre portable avec tête d'écoulement détachable

(30) Priority: 29.03.2007 GB 0706204
(43) Date of publication of application: 01.10.2008
(73) Proprietor: Vitalograph (Ireland) Limited, Ennis, Clare (IE)
(72) Inventor: Garbe, Bernhardt Rudolph, Buckingham MK 18BU (GB); Keane, Frank, Limerick (IE)
(74) Representative: Gallafent, Richard John

(56) References cited:
- WO-A-02/085207
- US-A- 5 816 246
- US-B1- 6 367 475

## Description

This invention relates to spirometers and in particular to compact hand-held spirometers for use in testing lung function.

In recent years, advances in microelectronics have enabled the production of hand-held spirometers. These consist basically of some form of mouthpiece or breathing tube into which the patient blows or on which the patient sucks, a mechanism in the breathing tube adapted to move when air flows through it, and an appropriate electronics and display package to detect and analyse the movement with a view to displaying useful results. A number of devices are known in the marketplace, for example the SpiroPro (Registered Trade Mark) pocket-sized spirometer produced by Jaeger-Toennies and the PulmoLife (Registered Trade Mark) from Micro Medical Limited. These both feature a spinning member which can be actuated by blowing through a mouthpiece fitted to a removable cylindrical "flow head" in which the spinning member is located.

A problem which arises with the use of such spirometers is one of potential contamination. Even though the use of a throwaway mouthpiece is customary with any spirometer, there is a material risk that the spirometer itself will become contaminated, even when using a mouthpiece, and that its subsequent use by another patient can lead to cross-infection. Although in the devices described above, the flow head can be removed from the body of the device and disinfected, the area around the flow head is not cleaned, and can be a source of cross-infection.

WO 2005/037102 seeks to avoid or at least reduce problems of contamination by providing a disposable mouthpiece unit which includes a freely opening rotatable member with a pair of opposed blades which is located between two deflector arrangements. The intention is to detect the rotation of the spinning member, for example using the blade to interrupt a beam of infrared light between an emitter and a sensor for such light. The signal produced from the infrared emitter/sensor combination can then be analysed and the result displayed.

The international publication referred to above gives no detail whatever as to how the disposable unit it describes is to cooperate with the remainder of the spirometer.

A problem which could be expected with the approach disclosed in this published international specification is that of maintaining consistency of operation. In order to produce a "throwaway" unit, it is necessary to produce it at extremely low cost, and the specification stresses this aspect and gives an indication of how it can be achieved. However, the processes for manufacturing throwaway disposable units as set out in WO 2005/037102 are not ones which are suitable for producing devices with consistent performance characteristics. In particular, slight dimensional variations will occur because of the plastics injection moulding technique and, over a period of time, systematic variation will occur because of mould wear.

US-A-5816246 discloses a hand-held spirometer construction with a removable flow chamber. The chamber surrounds a spring vane which may be rotated when air is inhaled or exhaled through the chamber.

US-B-6367475 discloses a peak respiratory flow monitoring unit where a flow head of conventional construction is a snap-fit on to a casing including a microprocessor, and also-including a pressure sensor which, when the flow head is in place, is used to measure the pressure in the flowhead.

However, in both of these constructions, the user still has to blow towards the main body of the unit and there is a risk of contamination of that body.

It is an object of the present invention to provide an improved arrangement of the flow head relative to the casing containing the main operative components of the spirometer, including the microprocessor and display components, with improved reduction in the risk of contamination while maintaining ease of use.

The invention is as defined in the independent claim 1.

According to the present invention, there is provided a portable hand-held spirometer apparatus consisting of a casing having a pair of opposed arms extending to one side thereon, and a flow head component including a channel through which air may flow, formations in the channel imparting a rotary movement to air flowing through the channel and a free-spinning vaned member mounted for rotation about an axis substantially coaxial with that of the channel, and wherein the other part of the casing has two open-ended recesses extending from the side of the other part of the casing to lie either side of the flow head component and dimensioned to receive the opposed arms when the two casing parts are fitted together, with the vaned member of the flow head lying between the two opposed arms.

By configuring the spirometer in this way, it is possible to operate using a number of detachable casing portions each incorporating a flow head, and each of which may be used once and then a batch of which may be cleaned and disinfected before re-use. Because they are re-usable, they may be made to high manufacturing tolerances ensuring continued accuracy of operation of the spirometer. The part of the casing incorporating the flow head may have, at an inlet end of the channel, an appropriate flange or ring for engagement with a conventional disposable spirometer mouthpiece containing an anti-bacterial filter, thereby to reduce the amount of material which may contaminate the flow head during use.

The location of the spinning vaned member between the two arms when the device is assembled for use enables the arms to carry single or multiple proximity or electromagnetic breaking-beam means enabling the rotation of the spinning vane unit to be observed and processed by appropriate circuitry in the casing. The results may be displayed using a suitable LCD display as used in other products of this type.

Preferably the casing part incorporating the flow head is manufactured from a few simple moulded pieces which are designed to snap fit together to produce a component which can be easily fitted on to the opposed arms and removed therefrom after use. Preferably the casing part including the flow head component consists of a pair of members each containing a portion of the flow passage and each of which has a central hub into which a rotatable vaned member may be fitted. Preferably the rotatable vaned member has a shaft, one end of which is fitted into each of the hubs on assembling the two halves of the component together. A transparent cylindrical wall section may be provided to enable the motion of the rotatable vaned member to be observed and measurements obtained. One or both of the clipped together members constituting the main body of the casing part may have a set of vanes therein designed to impart a peripheral rotational movement to air passing through the channel.

The present invention is illustrated in more detail with reference to a specific embodiment in various aspects in the accompanying drawings. In the drawings:
Figure 1 is a general arrangement view of a hand-held spirometer in accordance with the present invention;
Figure 2 is a view of the spirometer shown in Figure 1 with the part of the casing incorporating the flow head detached from the remainder of the unit;
Figure 3 is a diagrammatic exploded view of the major components which constitute the main casing portion and its conduits; and
Figures 4 and 5 are exploded views of the part of the casing incorporating the flow head.

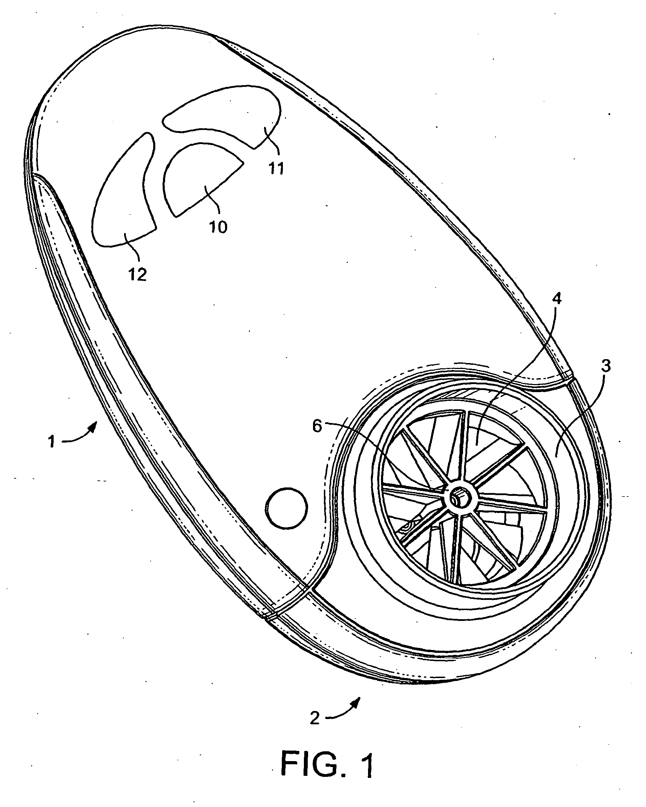

Referring first to Figure 1, this shows a hand-held spirometer in accordance with the present invention which consists basically of a main casing portion 1 and a casing portion 2 containing a flow head which is fitted on to main casing portion 1. As can be seen, the casing portion 2 has an annular upstanding flange 3 into which one end of a mouthpiece may be fitted.
Inside the flow head portion is a cylindrical passage through which the patient blows. Eight vanes 4 are located between the exterior cylindrical wall and a central hub 6. Air blown into the passage via a mouthpiece fitted into wall 3 is caused by vanes 4 to swirl peripherally as will be explained further below.

Also visible in Figure 1 are three actuation buttons 10, 11, 12 which are used to control the device in use.

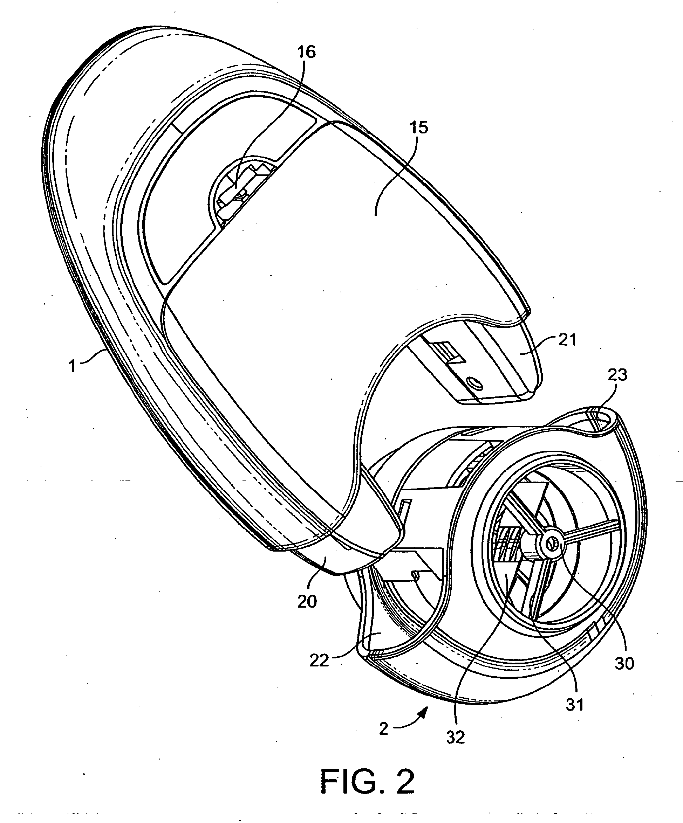

Figure 2 shows the device of Figure 1, but from the underside and with the casing portion 2 slightly separated from the main casing portion 1. Visible on the underneath of the main casing portion 1 is a cover 15 which conceals a pair of dry electrical cells located within it. A moulded spring catch 16 is provided in standard fashion to enable cover 15 to be removed and the cells replaced when they have run down.

As can be clearly seen from Figure 2, two arms 20, 21 project from the main casing portion 1 and these fit into corresponding apertures 22, 23 in the casing portion 2 incorporating the flow head when the two parts of the casing are assembled together.

Visible in Figure 2 is a downstream hub 30 supported by three radial bars 31 and also visible in Figure 2 is a rotatable vaned member 32.

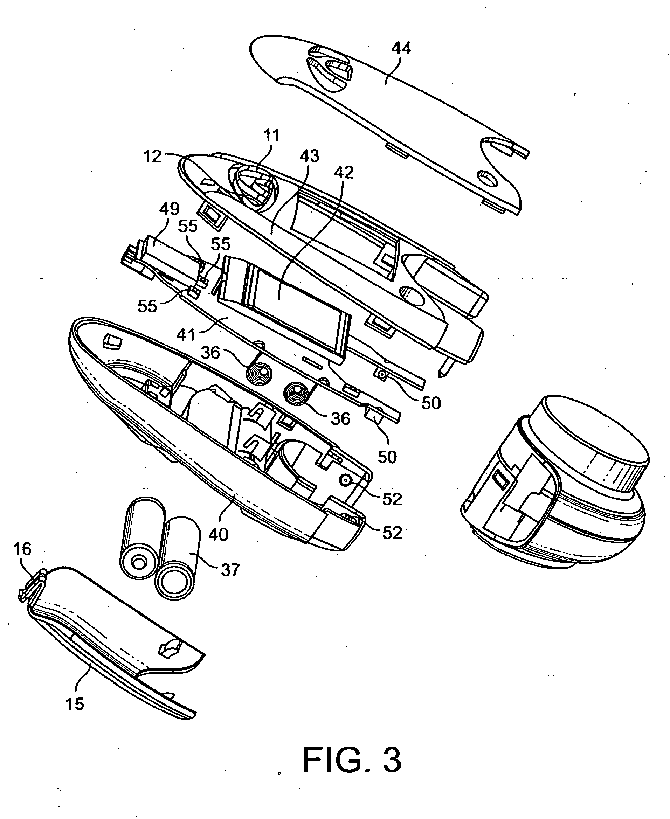

The construction of the main casing portion 1 of the spirometer is clearly illustrated in Figure 3 where its major components are shown in exploded view. The various components are mounted to a main moulding 40 into which cover 15 may be clipped. Body 40 has a couple of recesses for cells 37 which are arranged to contact resilient contacts 36 which are mounted on a main circuit board 41. Also mounted on circuit board 41 is an LCD display panel 42 and a dedicated programmed chip 49.

Board 41 also carries three pressure actuatable switches 55 which, when the unit is assembled together, can be actuated by pressing on buttons 10, 11 and 12. At one end, the board has a pair of projecting legs, each of which carries one of an infrared emitter and an infrared detector 50. When the components are assembled together, these register with apertures 52 in the main moulded body 40.

Circuit board 41 is assembled into the unit between member 40 and an upper moulding 43. The assembly is by click together resilient plastic tabs and stirrups in known fashion. A transparent cover 44 is a clip fit into moulded member 43.

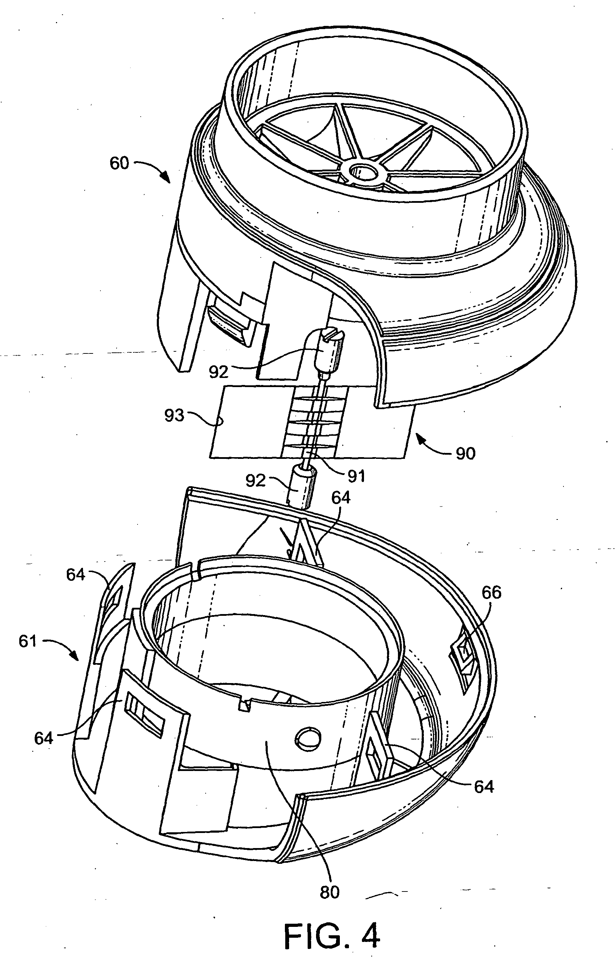

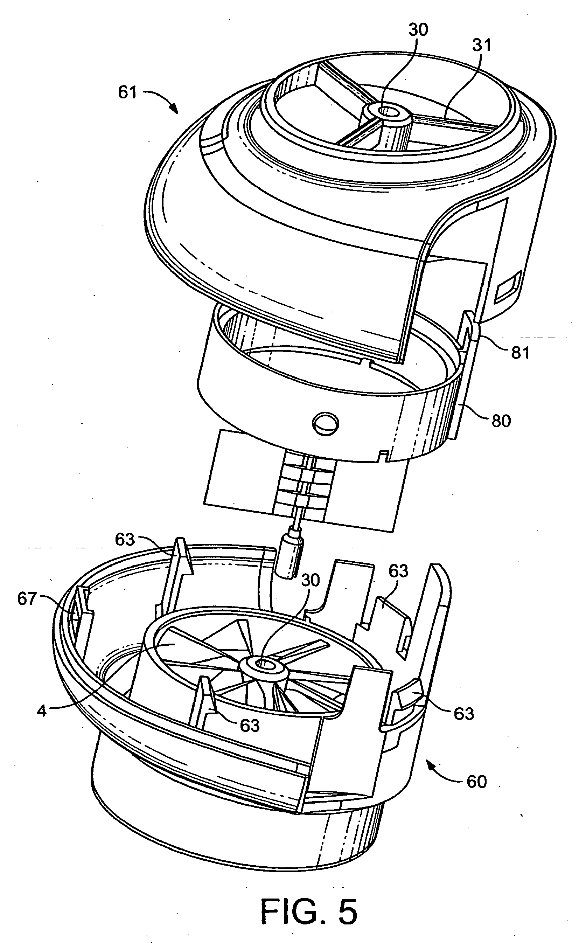

Referring now to Figures 4 and 5, the construction of the casing portion 2 incorporating the flow head can be clearly identified. It consists basically of two moulded plastics sections 60 and 61. Section 60 carries four resilient integrally moulded tabs 63, each of which is designed to fit into a rectangular aperture in an upstanding moulded post 64.

In addition to these four sets of inter-engaging members, the inner wall of member 61 bears a ramped tab 66 which is designed to engage in a slot 67 on an upstanding post forming part of moulded component 60.

Each of members 60 and 61 has a cylindrical throughflow passage in the centre of which there is an axial hub member 30. This is supported in member 61 by means of three spokes or bars 31 and in member 60 by means of the eight vanes 4.

Located between members 60 and 61 is a transparent cylindrical ring 80 which has an extending tab 81 with an aperture in it enabling it to be clipped over an appropriate projection moulded into component 61.

A vaned spinner member 90 is held captive when members 60 and 61 are assembled together with its vanes level with component 80 and with the ends of its shaft freely able to spin in hub 30. As can be seen in Figures 4 and 5, the shaft consists of a central narrow shaft 91 having two end pieces 92, each of which fits into hub 30 and set on shaft 91 a metal vane 93.

When the component shown in Figures 4 and 5 are all assembled together, they form the unit as shown in Figure 2 and, when this is pushed between the legs of the casing portion 1, the rotation of the rotary vane member 90 may be observed by means of the interruption of an infrared beam between emitter/sensor components 50, the beam passing through transparent member 80. The results of a patient blowing through a mouthpiece attached to flange 3 are analysed by circuitry of known type and displayed on display 42.

Once the patient has finished using the device, the two casing portions are simply pulled apart and the one incorporating the flow head can then be cleaned and disinfected. Any contamination on the outside of that part of the casing is thus removed, not just from the flow head itself.

## Claims

1. Portable hand-held spirometer apparatus including a first casing (1) housing electronics adapted to measure and display one or more lung function parameters and a detachable flow head unit (2) fitted thereto, the flow head unit (2) incorporating a flow head component including a channel through which air may flow, formations in the channel (4) imparting a rotary movement to air flowing through the channel and a free-spinning vaned member (90) mounted for rotation about an axis and **characterised in that** the said axis is substantially coaxial with that of the channel and **in that** the first casing (1) has a pair of arms (20, 21) extending to one side thereof, and the flow head unit (2) comprises a second casing incorporating two open-ended recesses (22, 23) extending from the side of the second casing (2) to a pair of positions located either side of the flow head component and dimensioned to receive the opposed arms (20, 21) when the two casings are fitted together, with the vaned member (90) of the flow head component lying between the two opposed arms (20, 21).

2. Spirometer apparatus according to Claim 1 wherein the flow head unit (2) has, at an inlet end of the channel, a flange or ring (3) for engagement with a conventional disposable spirometer mouthpiece containing an anti-bacterial filter.

3. Spirometer apparatus according to Claim 1 or 2 wherein the arms (20, 21) carry single or multiple proximity or electromagnetic breaking-beam means enabling the rotation of the free-spinning vaned member (90) to be observed and processed by appropriate circuitry in the first casing (1).

4. Spirometer apparatus according to Claim 3 wherein the first casing (1) houses an LCD display (42) to display the results deriving from the processing.

5. Spirometer apparatus according to any one of Claims 1 to 4 wherein the detachable flow head unit (2) is manufactured from a few simple moulded pieces (60, 61, 80) which snap fit together to produce a unit (2) which can be easily fitted on to the opposed arms (20, 21) and removed therefrom after use.

6. Spirometer apparatus according to any one of Claims 1 to 5 wherein the detachable flow head unit consists of a pair of members (60, 61), each containing a portion of the flow passage and each of which has a central hub (30) into which a rotatable vaned member (90) may be fitted.

7. Spirometer apparatus according to Claim 6 wherein the rotatable vaned member (90) has a shaft (91), one end of which is fitted into each of the hubs (30) on assembling the two halves of the flow head unit (2) together.

8. Spirometer apparatus according to Claim 6 or 7 and including a transparent cylindrical wall section (80) in a casing part adjacent the vaned member (90) to enable the motion of the rotatable vaned member (90) to be observed and measurements obtained.

9. Spirometer apparatus according to any one of Claims 6 to 8 wherein one or both of the clipped together members (60, 61) constituting the main body of the detachable flow head unit has a set of vanes (4) therein designed to impart a peripheral rotational movement to air flowing through the channel.

## Patentansprüche

1. Tragbare Handspirometervorrichtung, die ein erstes Gehäuse (1) enthält, in dem Elektronik untergebracht ist, die dafür ausgebildet ist, einen oder mehrere Lungenfunktionsparameter zu messen und anzuzeigen, sowie eine abnehmbare Strömungskopfeinheit (2) enthält, die an dem Gehäuse (1) angebracht ist, wobei die Strömungskopfeinheit (2) Folgendes enthält: eine Strömungskopfkomponente, die einen Kanal enthält, durch den Luft strömen kann; Ausbildungen in dem Kanal (4), die der durch den Kanal strömenden Luft einen Drall verleihen; und ein frei drehendes Flügelelement (90), das so montiert ist, dass es sich um eine Achse drehen kann; und
**dadurch gekennzeichnet, dass** diese Achse im Wesentlichen koaxial zu der Achse des Kanals verläuft, sowie **dadurch**, dass das erste Gehäuse (1) ein Paar Arme (20, 21) aufweist, die sich zu einer Seite des Gehäuses (1) erstrecken, und die Strömungskopfeinheit (2) ein zweites Gehäuse umfasst, das zwei offenendige Ausnehmungen (22, 23) enthält, die sich von der Seite des zweiten Gehäuses (2) zu einem Paar Positionen erstrecken, die sich auf jeweils einer Seite der Strömungskopfkomponente befinden und so bemessen sind, dass sie die gegenüberliegenden Arme (20, 21) aufnehmen können, wenn die zwei Gehäuse zusammengesetzt werden, wobei das Flügelelement (90) der Strömungskopfkomponente zwischen den beiden gegenüberliegenden Armen (20, 21) liegt.

2. Spirometervorrichtung gemäß Anspruch 1, wobei die Strömungskopfeinheit (2) an einem Einlassende des Kanals einen Flansch oder Ring (3) aufweist, um ein herkömmliches Einweg-Spirometermundstück, das einen antibakteriellen Filter enthält, in Eingriff zu nehmen.

3. Spirometervorrichtung gemäß Anspruch 1 oder 2, wobei die Arme (20, 21) ein einzelnes oder mehrere Näherungs- oder elektromagnetische strahlunterbrechende Mittel tragen, die es ermöglichen, die Drehung des frei drehenden Flügelelements (90) zu beobachten und durch geeignete Schaltkreise in dem ersten Gehäuse (1) zu verarbeiten.

4. Spirometervorrichtung gemäß Anspruch 3, wobei das erste Gehäuse (1) eine LCD-Anzeige (42) enthält, um die durch die Verarbeitung gewonnenen Ergebnisse anzuzeigen.

5. Spirometervorrichtung gemäß einem der Ansprüche 1 bis 4, wobei die abnehmbare Strömungskopfeinheit (2) aus einigen wenigen, simplen Formteilen (60, 61, 80) hergestellt ist, die sich zu einer Einheit (2) ineinander rasten lassen, die auf einfache Weise an den gegenüberliegenden Armen (20, 21) angebracht werden kann und nach Gebrauch von den gegenüberliegenden Armen (20, 21) abgenommen werden kann.

6. Spirometervorrichtung gemäß einem der Ansprüche 1 bis 5, wobei die abnehmbare Strömungskopfeinheit aus einem Paar Elementen (60, 61) besteht, die jeweils einen Abschnitt der Strömungspassage enthalten und von denen jedes eine mittige Nabe (30) aufweist, in die ein drehbares Flügelelement (90) eingesetzt werden kann.

7. Spirometervorrichtung gemäß Anspruch 6, wobei das drehbare Flügelelement (90) eine Welle (91) aufweist, von der - beim Zusammensetzen der beiden Hälften der Strömungskopfeinheit (2) - ein Ende in jede der Naben (30) eingesetzt wird.

8. Spirometervorrichtung gemäß Anspruch 6 oder 7, die einen transparenten zylindrischen Wandabschnitt (80) in einem Gehäuseteil neben dem Flügelelement (90) enthält, damit die Bewegung des drehbaren Flügelelements (90) beobachtet und Messwerte erhalten werden können.

9. Spirometervorrichtung gemäß einem der Ansprüche 6 bis 8, wobei sich in einem oder in beiden der ineinander gerasteten Elemente (60, 61), die den Hauptkörper der abnehmbaren Strömungskopfeinheit bilden, ein Satz Leitschaufeln (4) befindet, die dafür ausgelegt sind, Luft, die durch den Kanal strömt, einen Drall zu verleihen.

## Revendications

1. Spiromètre portatif comprenant un premier boîtier (1) logeant l'électronique adaptée pour mesurer et afficher un ou plus paramètres de la fonction pulmonaire et une unité de tête d'écoulement détachable (2) qui y est intégrée, l'unité de tête d'écoulement (2) incorporant un élément de tête d'écoulement comprenant un canal à travers lequel l'air peut s'écouler, des formations dans le canal (4) transmettant un mouvement de rotation à l'air s'écoulant à travers le canal et une roue à pales (90) libre en rotation montée de façon rotative autour d'un axe, **caractérisé en ce que** ledit axe est sensiblement coaxial avec celui du canal et **en ce que** le premier logement (1) présente une paire de bras (20, 21) s'y étendant d'un côté, et l'unité de tête d'écoulement (2) comprend un deuxième boîtier incorporant deux renfoncements ouverts (22, 23) s'étendant depuis un côté du second boîtier (2) vers une paire de positions situées de chaque côté de l'élément de la tête d'écoulement et dimensionnées pour recevoir les bras (20, 21) opposés lorsque les deux boîtiers sont mis ensembles, avec la roue à pales (90) de l'élément de la tête d'écoulement disposée entre les deux bras opposés (20, 21).

2. Spiromètre portatif selon la revendication 1, dans lequel l'unité de tête d'écoulement (2) présent, sur une arrivé du canal, une collerette ou un anneau pour s'engager avec un embout jetable traditionnel de spiromètre contenant un filtre anti-bactérien.

3. Spiromètre portatif selon la revendication 1 ou 2, dans lequel les bras (20, 21) portent un moyen d'interruption de faisceau multiple ou unique, électromagnétique ou par proximité, permettant l'observation et le traitement par un circuit approprié du premier boîtier (1) de la rotation de la roue à pales (90) libre en rotation.

4. Spiromètre portatif selon la revendication 3, dans lequel le premier boîtier (1) loge un afficheur à cristaux liquides (42) destiné à afficher les résultats provenant du traitement.

5. Spiromètre portatif selon l'une quelconque des revendications 1 à 4, dans lequel l'unité de tête d'écoulement détachable (2) est fabriquée à partir de quelques pièces moulées simples (60, 61, 80) qui vont ensemble par encliquetage pour produire une unité (2) qui peut être facilement monté sur les bras opposés (20, 21) et en être retiré après utilisation.

6. Spiromètre portatif selon l'une quelconque des revendications 1 à 5, dans lequel l'unité de tête d'écoulement détachable (2) comprend une paire d'organes (60, 61), chacun contenant une partie du passage d'écoulement et chacun d'eux ayant un moyeu central (30) dans lequel une roue à pales rotative (90) peut être montée.

7. Spiromètre portatif selon la revendication 6, dans lequel la roue à pales rotative (90) présent un axe (91), un bout duquel rentre dans chacun des moyeux (30) de l'assemblage des deux moitiés de l'unité de tête d'écoulement (2) ensemble.

8. Spiromètre portatif selon la revendication 6 ou 7 et comprenant une section de paroi cylindrique transparente dans une partie de boîtier adjacente à la roue à pales rotative (90) afin d'autoriser l'observation du mouvement de la roue à pales (90) rotative et l'acquisition de mesures.

9. Spiromètre portatif selon l'une quelconque des revendication 6 à 8, dans lequel un ou deux des organes (60, 61) attachés ensembles constituant le corps principal de l'unité de tête d'écoulement détachable y ont un ensemble d'aubes (4) conçues pour communiquer un mouvement de rotation périphérique à l'air passant à travers le canal.
